# EUROPEAN PATENT APPLICATION

(11) **EP 2 532 318 A2**
(43) Date of publication of application: **12.12.2012**
(21) Application number: 12183604.3
(22) Date of filing: 30.04.2009
(51) Int. Cl.: A61B 17/88, A61B 17/58, A61F 2/00, A61F 2/46, A61B 17/00

(54) **Sheaths for extra-articular implantable systems**

(30) Priority: 30.04.2008 US 49421 P; 30.04.2008 US 113188; 30.04.2008 US 113186
(62) Divisional of application: 09739227.8
(71) Applicant: Moximed, Inc., Hayward, CA 94545 (US)
(72) Inventor: Makower, Joshua, Los Altos, CA California 94022 (US); Clifford, Anton G., Mountain View, CA California 94040 (US); Friedmann, Josef L, Scotts Valley, CA California 95066 (US); Slone, Clinton N., San Francisco, CA California 94114 (US); Regala, Alan C., Mountain View, CA California 94041 (US); Landry, Michael E., Austin, TX Texas 78746 (US); Gabriel, Stefan, Mattapoisett, MA Massachusetts 02739 (US); O'Conell, Mary, Menlo Park, CA California 94025 (US)
(74) Representative: Jansen, Cornelis Marinus

(57) **Abstract**

Various embodiments are directed to a sheath for covering one or more components of an extra-articular implantable mechanical energy absorbing system. The sheath is generally an elongated structure having an inner space extending the length thereof. In use, the sheath can exclude the energy absorbing system from surrounding tissue and facilitate creating a capsule for its operation. Materials and dimensions are selected to achieve these purposes. The ends of the sheath include various attachment mechanisms for securing the sheath to one or more components of an extra-articular implantable mechanical energy absorbing system.

## Description

### FIELD OF EMBODIMENTS

Various embodiments disclosed herein are directed to structures for attachment to body anatomy, and more particularly, towards approaches for providing a protective sheath for extra-articular implantable systems.

### BACKGROUND

Joint replacement is one of the most common and successful operations in modern orthopaedic surgery. It consists of replacing painful, arthritic, worn or diseased parts of a joint with artificial surfaces shaped in such a way as to allow joint movement. Osteoarthritis is a common diagnosis leading to joint replacement. Such procedures are a last resort treatment as they are highly invasive and require substantial periods of recovery. Total joint replacement, also known as total joint arthroplasty, is a procedure in which all articular surfaces at a joint are replaced. This contrasts with hemiarthroplasty (half arthroplasty) in which only one bone's articular surface at a joint is replaced and unicompartmental arthroplasty in which the articular surfaces of only one of multiple compartments at a joint (such as the surfaces of the thigh and shin bones on just the inner side or just the outer side at the knee) are replaced. Arthroplasty as a general term, is an orthopaedic procedure which surgically alters the natural joint in some way. This includes procedures in which the arthritic or dysfunctional joint surface is replaced with something else, procedures which are undertaken to reshape or realigning the joint by osteotomy or some other procedure. As with joint replacement, these other arthroplasty procedures are also characterized by relatively long recovery times and their highly invasive procedures. A previously popular form of arthroplasty was interpositional arthroplasty in which the joint was surgically altered by insertion of some other tissue like skin, muscle or tendon within the articular space to keep inflammatory surfaces apart. Another previously done arthroplasty was excisional arthroplasty in which articular surfaces were removed leaving scar tissue to fill in the gap. Among other types of arthroplasty are resection(al) arthroplasty, resurfacing arthroplasty, mold arthroplasty, cup arthroplasty, silicone replacement arthroplasty, and osteotomy to affect joint alignment or restore or modify joint congruity. When it is successful, arthroplasty results in new joint surfaces which serve the same function in the joint as did the surfaces that were removed. Any chondrocytes (cells that control the creation and maintenance of articular joint surfaces), however, are either removed as part of the arthroplasty, or left to contend with the resulting joint anatomy. Because of this, none of these currently available therapies are chondro-protective.

A widely-applied type of osteotomy is one'in which bones are surgically cut to improve alignment. A misalignment due to injury or disease in a joint relative to the direction of load can result in an imbalance of forces and pain in the affected joint. The goal of osteotomy is to surgically re-align the bones at a joint and thereby relieve pain by equalizing forces across the joint. This can also increase the lifespan of the joint. When addressing osteoarthritis in the knee joint, this procedure involves surgical re-alignment of the joint by cutting and reattaching part of one of the bones at the knee to change the joint alignment, and this procedure is often used in younger, more active or heavier patients. Most often, high tibial osteotomy (HTO) (the surgical re-alignment of the upper end of the shin bone (tibia) to address knee malalignment) is the osteotomy procedure done to address osteoarthritis and it often results in a decrease in pain and improved function. However, HTO does not address ligamentous instability - only mechanical alignment. HTO is associated with good early results, but results deteriorate over time.

Other approaches to treating osteoarthritis involve an analysis of loads which exist at a joint. Both cartilage and bone are living tissues that respond and adapt to the loads they experience. Within a nominal range of loading, bone and cartilage remain healthy and viable. If the load falls below the nominal range for extended periods of time, bone and cartilage can become softer and weaker (atrophy). If the load rises above the nominal level for extended periods of time, bone can become stiffer and stronger (hypertrophy). Finally, if the load rises too high, then abrupt failure of bone, cartilage and other tissues can result. Accordingly, it has been concluded that the treatment of osteoarthritis and other bone and cartilage conditions is severely hampered when a surgeon is not able to precisely control and prescribe the levels of joint load. Furthermore, bone healing research has shown that some mechanical stimulation can enhance the healing response and it is likely that the optimum regime for a cartilage/bone graft or construct will involve different levels of load over time, e.g. during a particular treatment schedule. Thus, there is a need for devices which facilitate the control of load on a joint undergoing treatment or therapy, to thereby enable use of the joint within a healthy loading zone.

Certain other approaches to treating osteoarthritis contemplate external devices such as braces or fixators which attempt to control the motion of the bones at a joint or apply cross-loads at a joint to shift load from one side of the joint to the other. A number of these approaches have had some success in alleviating pain but have ultimately been unsuccessful due to lack of patient compliance or the inability of the devices to facilitate and support the natural motion and function of the diseased joint. The loads acting at any given joint and the motions of the bones at that joint are unique to the body that the joint is a part of. For this reason, any proposed treatment based on those loads and motions must account for this variability to be universally successful. The mechanical approaches to treating osteoarthritis have not taken this into account and have consequently had limited success.

Prior approaches to treating osteoarthritis have also failed to account for all of the basic functions of the various structures of a joint in combination with its unique movement. In addition to addressing the loads and motions at a joint, an ultimately successful approach must also acknowledge the dampening and energy absorption functions of the anatomy, and be implantable via a minimally invasive technique. Prior devices designed to reduce the load transferred by the natural joint typically incorporate relatively rigid constructs that are incompressible. Mechanical energy (E) is the action of a force (F) through a distance (s) (i.e., E=F ^{x}s). Device constructs which are relatively rigid do not allow substantial energy storage as the forces acting on them do not produce substantial deformations - do not act through substantial distances - within them. For these relatively rigid constructs, energy is transferred rather than stored or absorbed relative to a joint. By contrast, the natural joint is a construct comprised of elements of different compliance characteristics such as bone, cartilage, synovial fluid, muscles, tendons, ligaments, etc. as described above. These dynamic elements include relatively compliant ones (ligaments, tendons, fluid, cartilage) which allow for substantial energy absorption and storage, and relatively stiffer ones (bone) that allow for efficient energy transfer. The cartilage in a joint compresses under applied force and the resultant force displacement product represents the energy absorbed by cartilage. The fluid content of cartilage also acts to stiffen its response to load applied quickly and dampen its response to loads applied slowly. In this way, cartilage acts to absorb and store, as well as to dissipate energy.

With the foregoing applications in mind, it has been found to be necessary to develop effective structures for mounting to body anatomy. Such structures should conform to body anatomy and cooperate with body anatomy to achieve desired load reduction, energy storage, and energy transfer. These structures should include mounting means for attachment of complementary structures across articulating joints.

For these implant structures to function optimally, they must not cause an adverse disturbance to apposing tissue in the body, nor should their function be affected by anatomical tissue and structures impinging on them. Therefore, what is needed is an approach which addresses both joint movement and varying loads as well as complements underlying anatomy and provides an effective protective sheath for an implantable, articulating assembly.

### SUMMARY

Briefly, and in general terms, various embodiments are directed to sheaths for covering one or more components used in connection with extra-articular implantable systems. According to one embodiment, the sheath includes a material for housing the extra-articular implantable system without interfering with the system's function and protecting the surrounding body tissues from the movement of the system. The sheath can have structure that attaches the first end of the sheath to a first component of the extra-articular implantable system. The sheath also can have structure that attaches the second end to a second component of the extra-articular implantable system.

In various disclosed embodiments, the sheath prevents impingement of surrounding tissue within structure defining an energy absorbing system. Moreover, the sheath facilitates the removability and replaceability of an energy absorbing component of an extra-articular implantable mechanical energy absorbing system. In this regard, the sheath can be configured to create a pseudo-capsule within a patient's body for the moving elements of the energy absorbing system. One contemplated approach involves the sheath moving with the surrounding tissue, but the energy absorbing component is excluded from such motion. Accordingly, the sheath protects the absorbing component from tissue ingrowth. In one particular embodiment, expanded polytetrafluoroethylene (ePTFE) is employed as a material for the sheath. Such material has been found to have similar responses as natural tissue in areas such as elasticity and conformability. Moreover, various shapes and thicknesses of sheaths are contemplated as well as approaches to connecting the sheaths to the energy absorbing system. Additionally, the sheaths can include multiple layers having different physical properties. For example, a sheath is composed of an outer layer promoting tissue ingrowth and an inner layer having lubricious properties. Optionally, the outer surface of the sheaths may be coated, impregnated, or otherwise include one or more compositions that inhibit or promote tissue ingrowth.

According to one embodiment, the sheath is a generally cylindrical tube of ePTFE having reinforced areas at the ends of the sheath. The reinforced areas provide a tougher, low-profile area on the sheath for securing the sheath to a component of the extra-articular implantable mechanical energy absorbing system. In one embodiment, the reinforced areas are formed by sintering (i.e., applying heat and pressure) a piece of material such as ePTFE or PTFE to the end of the sheath. It is contemplated that the reinforced area may be any size, shape, or thickness. The reinforced area also includes one or more openings sized to receive one or more fastening members. Optionally, the ends of the sheath are cut at an angle so that the sheath contours to the component of the system thereby minimizing the overall profile of the sheath on the extra-articular implantable mechanical energy absorbing system.

In another embodiment, the sheath includes an elongated tube having an inner diameter, an outer diameter, and a first end opposite a second end. The sheath also includes a snap ring embedded in the first and second ends of the elongate body. The snap ring has a main body and a plurality of hooks extending from the main body. The snap ring is sized to be coupled around a portion of the base component, with the hooks engaging one or more features on the base component, thereby securing the sheath to the extra-articular implantable mechanical energy absorbing system.

In addition to sheaths, various embodiments are directed to a covering attachable to a surface of a base component. According to one embodiment, the covering includes a body having an upper surface, a lower surface, and a perimeter. The body is shaped to cover an upper surface of a base component. The body also includes one or more coupling features provided about the perimeter of the body, wherein the coupling structures secure the body to the base component. In yet a further approach, the sheath includes protective covering extensions which can be configured about a length of a base component.

Various approaches are also contemplated for attaching the sheath to components of an energy manipulating system. Certain of the approaches lend themselves to both easy and convenient attachment as well as removal from an interventional site. In one contemplated approach, an energy manipulating system is at the outset provided with a sheath configured thereabout, the complete assembly readied for implantation at an interventional site.

Other features of the present disclosure will become apparent from the following detailed description, taken in conjunction with the accompanying drawings, which illustrate, by way of example, the principles of the approach.

### BRIEF DESCRIPTION OF THE DRAWINGS

FIG. 1A is a side view of one embodiment of a sheath.
FIG. 1B is a top plan view of the sheath of FIG. 1A.
FIG. 1C is a side perspective view of the sheath of FIG. 1A covering components of an extra-articular implantable mechanical energy absorbing system.
FIG. 1D is an enlarged view of one end of the sheath shown in FIG. 1C.
FIG. 2A is a side view of another embodiment of a sheath.
FIG. 2B is an end view of the sheath of FIG. 2A in a closed position.
FIG. 2C is an end view of the sheath of FIG. 2A in an opened position.
FIG. 3 is a side view of yet another embodiment of a sheath.
FIG. 4A is a side view of another embodiment of a sheath covering portions of an extra-articular implantable mechanical energy absorbing system, wherein the system is in a first position within the sheath.
FIG. 4B is a side view of the sheath of FIG. 4A, wherein the system is in a second position within the sheath.
FIG. 5A is a side view of another embodiment of a sheath coupled to a base component of an extra-articular implantable mechanical energy absorbing system.
FIG. 5B is an end view of the sheath of FIG. 5A.
FIGS. 6A-D are cross-sectional views of various additional embodiments of a sheath.
FIG. 7 is a perspective view of one embodiment of a sheath having two layers.
FIG. 8 is a side view of one embodiment of a sheath having internal reinforcement.
FIG. 9 is a side view of one embodiment of a sheath having variable wall thicknesses.
FIG. 10 is a side view of one embodiment of a sheath having a zone of flexion.
FIG. 11A is a side view of another embodiment of a sheath having a zone of flexion.
FIG. 11B is a side view of the sheath of FIG. 11A in a flexed configuration.
FIG. 12 is a perspective view of one embodiment of a fastening member for securing a sheath to a component of an extra-articular implantable mechanical energy absorbing system.
FIG. 13A is a side view of one embodiment of a tool used to secure the fastening member of FIG. 12 onto a component of an extra-articular implantable mechanical energy absorbing system.
FIG. 13B is an enlarged view of an end of the tool of FIG. 13A in a first position.
FIG. 13C is an enlarged view of an end of the tool of FIG. 13A in a second position.
FIG. 14 is a side perspective view of one embodiment of a sheath coupled to a base component.
FIG. 15 is a side perspective view of another embodiment of a sheath coupled to a base component.
FIG. 16 is a side perspective view of yet another embodiment of a sheath coupled to a base component.
FIG. 17A is a side perspective view of another embodiment of a sheath coupled to a base component.
FIG. 17B is a side view of one embodiment of a snap used to couple the sheath of FIG. 17A to a base component.
FIG. 17C is a back view of another embodiment of a snap used to couple the sheath of FIG. 17A to a base component.
FIG. 18A-18D illustrate various embodiments of a clip used to couple a sheath to a base component.
FIG. 19A is a side perspective view of another embodiment of a sheath coupled to a base component.
FIG. 19B is a side view of the sheath of FIG. 19A.
FIG. 20A is a side view of one embodiment of a sheath having an attachment structure.
FIG. 20B is a side view of another embodiment of a sheath having an attachment structure.
FIG. 21 is a side view of a further embodiment of a sheath having an attachment structure.
FIG. 22 is a side view of yet a further embodiment of a sheath having an attachment structure.
FIG. 23 is a side view of another embodiment of a sheath having an attachment structure.
FIG. 24 is a side view of an additional embodiment of a sheath having an attachment structure.
FIG. 25A is a perspective view of one embodiment of a sheath having an attachment structure.
FIG. 25B is a partially exploded view of the sheath of FIG. 25A.
FIG. 26A is a side view of one embodiment of a sheath having an attachment structure.
FIG. 26B is an end view of the sheath of FIG. 26A, wherein the attachment structure is shown in an open position.
FIG. 26C is an end view of the sheath of FIG. 26A, wherein the attachment structure is shown in a semi-closed position.
FIG. 26D is an end view of the sheath of FIG. 26A, wherein the attachment structure is shown in a closed position.
FIG. 27A is a perspective view of one embodiment of a protective covering that is attachable to a base component.
FIG. 27B is a side view of a fastening structure for the protective covering of FIG. 27A.
FIG. 27C is a cross-sectional view of a fastening structure for the protective covering of FIG. 27A.
FIG. 27D is a side view of a protective covering of FIG. 27C coupled to a base component.
FIG. 27E is a perspective view of one embodiment of a protective covering that is attachable to a base component.
FIG. 27F is a cross-sectional view of a fastening structure for a protective covering.
FIG. 27G is a top view of one embodiment of a protective covering having a fastening structure.
FIG. 27H is a cross-sectional view of the edge of the protective covering shown in FIG. 27G.
FIG. 28A is an exploded perspective view of one embodiment of a protective covering that is attachable to a base component.
FIG. 28B is a cross-section view of the protective covering of FIG. 28A coupled to the base component.
FIG. 29 is a perspective view, depicting a sheath and protective covering assembly.

### DETAILED DESCRIPTION

The present disclosure is directed towards various embodiments of sheaths for covering one or more components of an extra-articular implantable system. Generally, the implantable system is composed of a link that spans a joint (e.g., knee, elbow, finger, toe) and manipulates forces experienced by the joint. The ends of the link are coupled to mounts that allow the absorber to track the natural movement of joint. The mounts are secured to base components that are fixed to the bones adjacent to the joint. In preferred embodiments, the sheath covers a mechanical energy absorbing link coupled to the mounts with articulating members such as a ball-and-socket, pivot or universal joint connection.

According to one embodiment, the sheath is an elongated tube having an inner passage or space extending the length of the elongated tube. The sheath includes various attachment mechanisms for securing the sheath to the mounting member or base component. In one embodiment, the sheath promotes the formation of a fibrous capsule around the implanted system thereby isolating the device from surrounding body structure. Alternatively, the sheath includes (or is made from) material that promotes tissue ingrowth. In either embodiment, the sheath isolates the mobile elements of the implanted system from surrounding tissues and prevents tissue adhesions to components of the implanted system. As a result, tissue impingement on the components of the implanted system is minimized thereby facilitating the replacement of the various components of the extra-articular implantable mechanical energy absorbing system.

It has been found that in certain situations, adjustments to an implanted energy absorbing or manipulating system are necessary. In other scenarios, it may be necessary or beneficial to remove the implanted system from the interventional site. Accordingly, the capsule the sheath provides about the implanted system aids in accomplishing adjustments or completed removal of the system. That is, the capsule created by the sheath provides a convenient space for accessing the energy manipulating system contained within the sheath.

Other embodiments are directed to a covering attached to the outer surface of the base component. The covering is attached to the base component via one or more hooks, snap fittings, or the like. The covering is used to provide padding in those instances where the base component is mounted to a bone that does not have much overlying connective or fatty tissue, for example the tibia. Additionally, the protective covering improves the aesthetic appearance of the base component through the skin to give the base component a tapered appearance.

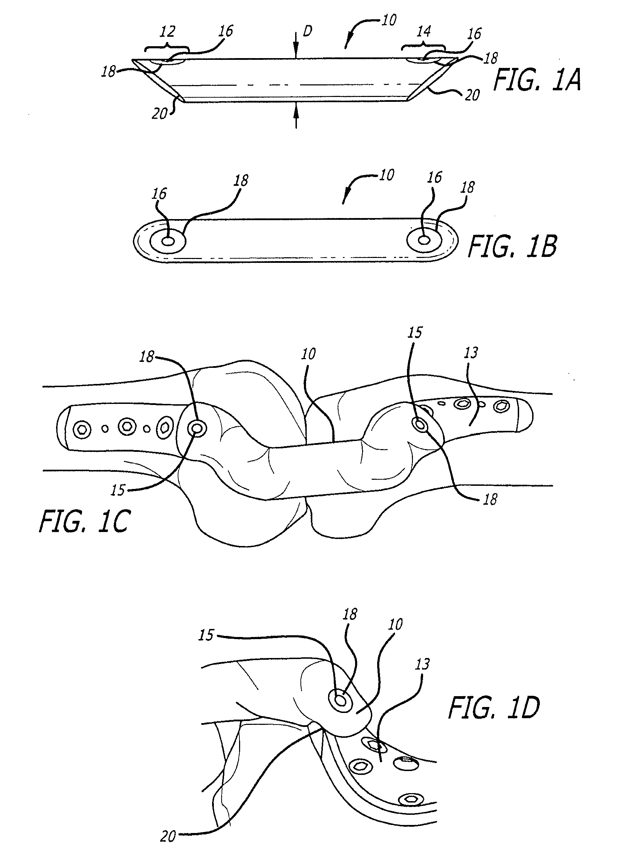

Referring now to the drawings, wherein like reference numerals denote like or corresponding parts throughout the drawings and, more particularly to FIGS. 1A-27H, there are shown various embodiments of a sheath 10 for enveloping one or more components of an extra-articular implantable system. The various contemplated shapes of the sheaths are first described followed later by descriptions of materials and coatings as well as approaches to their attachment within the interventional site. It is to be recognized that each of the described aspects maybe incorporated into any one of the disclosed sheath embodiments to create structure best suited for a particular purpose.

As shown in FIGS. 1A-B, in a preferred approach, the sheath 10 is an elongated tube having a first end 12 opposite a second end 14. The sheath 10 includes an inner bore that is sized to envelop one or more components of an extra-articular implantable mechanical energy absorbing system. As shown in FIG. IA, the sheath 10 is a generally cylindrical tube having angled ends, wherein the top surface of the sheath is longer than the bottom surface of the sheath. Alternatively, the sheath (not shown) has squared-off ends whereby the ends of the sheath are substantially perpendicular to the longitudinal axis of the sheath. FIGS. 1C-D depict the attachment of the sheath 10 to a component 13 of the implanted system with a fastening member 15 (Described in more detail below).

As shown in FIGS. 1C and D, the sheath generally conforms to the underlying shape of the implanted energy absorbing system and protects the implanted system from surrounding tissue. In this way, the implanted energy absorbing system is substantially or completely excluded from tissue ingrowth and can operate unimpeded and as intended. The sheath 10 also provides an outer profile well suited for exhibiting a natural appearance and feel under and through a patient's skin.

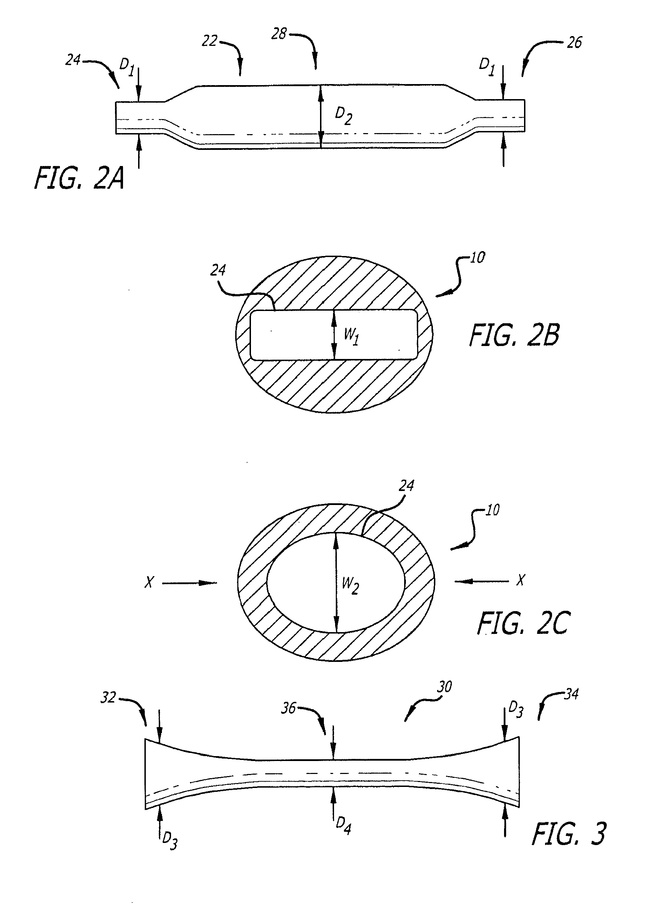

Generally, the inner diameter of the sheath is dimensioned off the enveloped implanted energy absorbing component such that there is approximately 1 mm of clearance between the sheath and the component. In one embodiment, the sheath has an inner diameter of approximately 14.5 mm. The inner diameter of the sheath (or diameter of the inner bore) is the same along the entire length of the sheath. In another embodiment, the inner diameter D1 at the ends 24, 26 of the sheath 22 is smaller than the inner diameter D2 at the middle 28 of the sheath. For example, the ends of the sheath tapers to a smaller inner diameter as shown in FIG. 2A. In yet another embodiment, the inner diameter D3 at the ends 32, 34 of the sheath 30 is larger than the inner diameter D4 at the middle 36 of the sheath as shown in FIG. 3.

Referring to FIGS. 2A-C, the sheath 22 can form an elongated body having tapered ends. As shown in FIG. 2B, the tapered end 24 has a generally rectangular opening. In alternate embodiments, the opening may be elliptical or oval shaped. In certain applications, the tapered, shaped ends provide better fit and/or transition to one or more components of the extra-articular implantable mechanical energy absorbing system. According to one embodiment, the shaped ends include a super-elastic member (not shown) embedded therein. FIG. 2B illustrates an end view of one shaped end 24 in a first configuration having a first diameter D5. A force X is applied to opposite sides of the shaped end 24 that causes the shaped end to alter from a first configuration to a second configuration. In the second configuration, as shown in FIG. 2C, the width W2 of the opening on the shaped end 24 in the second configuration is greater than the first width W1 in the first configuration. In the second configuration, an end-user (e.g., surgeon) may adjust the location of the shaped end on the base component (or other component of implanted system). When force X is removed, the shaped end 24 returns to the first configuration and engages the surfaces of the base component (or other component of implanted system).

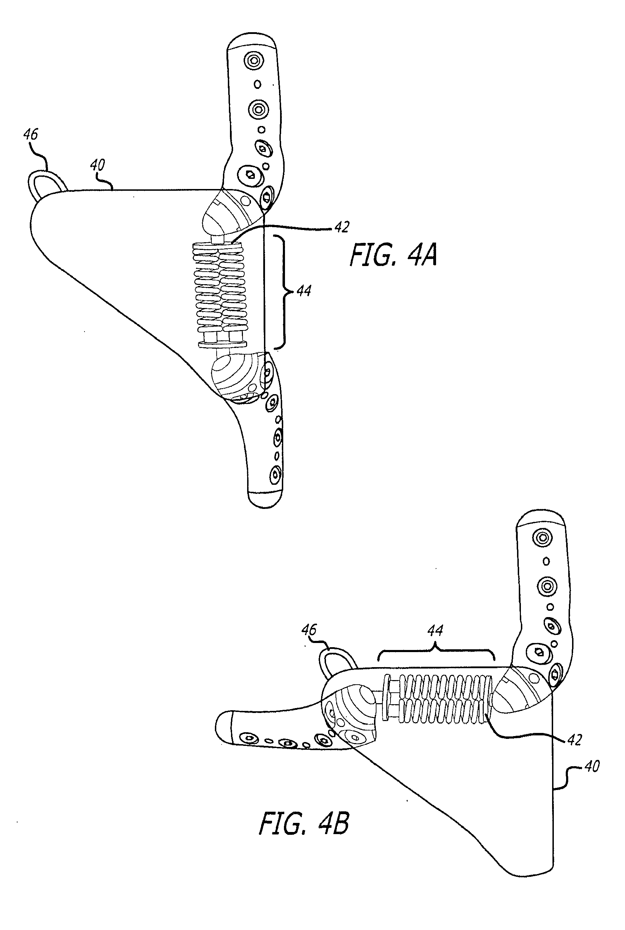

FIGS. 4A-B illustrate another embodiment of a sheath 40 for an extra-articular implantable mechanical energy absorbing system. Rather than conforming closely to the contour of the energy absorbing system, the sheath 40 defines a channel in which one or more components 42 of the extra-articular implantable mechanical energy absorbing system 44 may move through. That is, the sheath 40 encloses the full range of motion of the energy absorbing system 44 from full extension as shown in FIG. 4A to full flexion as shown in FIG. 4B, for example when the energy absorbing system is mounted along side of and outside the capsule of the knee joint. Optionally, as shown in FIGS. 4A-B, the sheath 40 includes a loop 46 for securing the posterior end of the sheath to a bone or other surrounding tissue. In yet another embodiment, one or more components 42 of the system 44 are enveloped by a first sheath (e.g., a sheath shown in FIGS. 1A-2B) and the covered components are placed within a second sheath such as the sheath 40 shown in FIG. 4A.

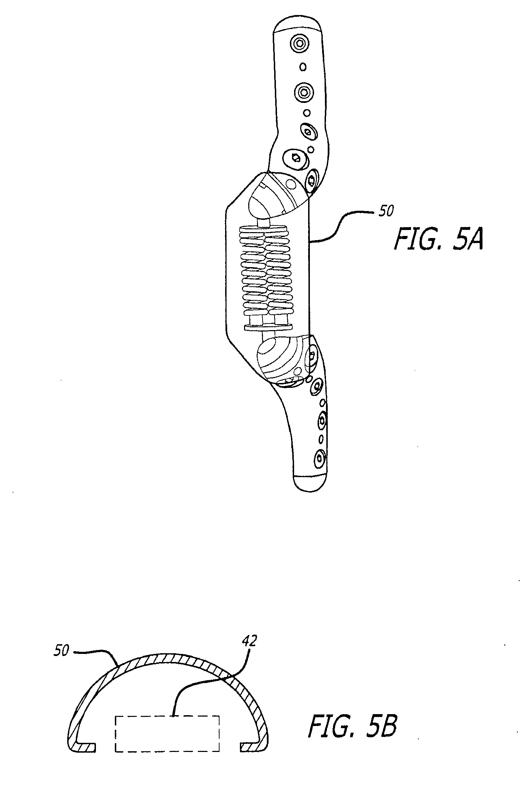

FIGS. 5A-B illustrate yet another embodiment of a sheath 50. In this embodiment, the sheath 50 is a canopy that covers the external components 42 of the implanted system 44 but leave uncovered portions of the implant configured adjacent anatomy which the implant is affixed. As shown in FIG. 5B, the sheath 50 is shaped or molded to have a curved body to cover one or more components 42 of the implanted system 44. In another embodiment, the sheath is a pliable sheet of material that is bent around the implanted system 44 and secured to one or more components 42 of the implanted system.

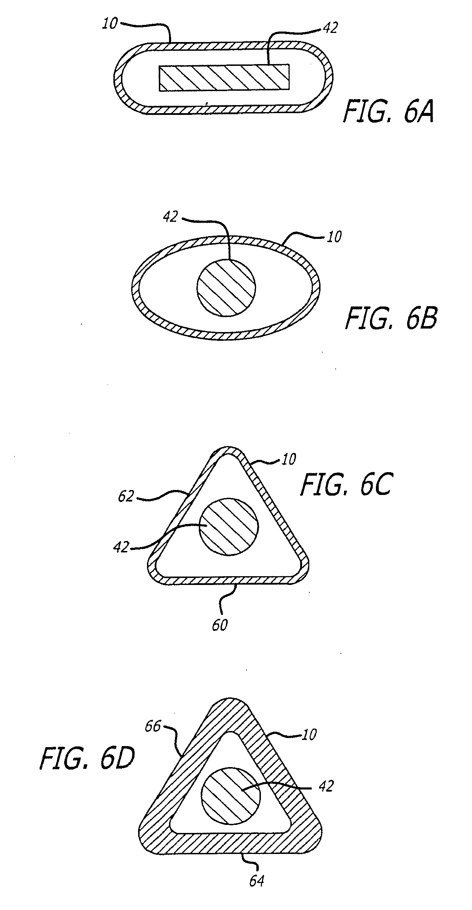

Whereas the sheath can be conformable and unconstrained, the various embodiments of the disclosed sheath may have different cross-sections. In one embodiment, the sheath 10 has a generally flattened tubular cross-section as shown in FIG. 6A. In another embodiment, the sheath 10 has an ovoid cross-section as shown in FIG. 6B. In another embodiment, the sheath can embody a simple circular cross-section. In yet another embodiment, the sheath 10 has an irregularly-shaped cross-section similar to a dome as shown in FIG. 6C. The dome-shaped sheath 10 has generally flat area 60 that would be configured closest to the surface of the bone or other underlying structure to which the implant is attached, and a curved area 62 that is positioned closest to overlying tissue. In the embodiment depicted in FIG. 6D, the sheath surface 64 closest to the bone surface is thinner than the portion of the sheath surface 66 that is closer to the skin surface. The thicker portion 66 of the sheath 10 provides padding for the implanted system 44. The padding also provides better aesthetics by minimizing the appearance of the edges or other rigid surfaces of the implanted system through the skin. Such variance in thickness can be incorporated into any one of the disclosed embodiments.

The various embodiments of the sheath, as shown in FIGS. 1A-5B and in applications relative to the knee joint, are generally 100 mm in length. In one embodiment, the sheath is longitudinally compressed to about 80 mm in length. The pre-compression of the sheath provides about 20 mm of displacement (100 mm length - 80 mm pre-compressed length) to compensate for the lengthening of the extra-articular implantable mechanical energy absorbing system during articulation of members defining a joint. In other embodiments, the sheath will have a length to suit the particular application of the extra-articular implantable mechanical energy absorbing system (e.g., finger, toe or elbow). Optionally, in another embodiment, the sheath is not pre-compressed prior to use.

Additionally, the disclosed sheaths may have a uniform wall thickness. According to one embodiment, the sheath 10 has a wall thickness of approximately 0.6 mm throughout the entire length of the sheath. In other embodiments, the sheath has a wall thickness ranging from approximately 0.5 mm to 1.0 mm. In yet another embodiment, the sheath has areas of variable thickness: The thickness of the wall is varied based upon the wear requirements, the desired cosmesis effect, and location of use within the body.

Moreover, the various embodiments of the disclosed sheaths shown in the previous figures as well as those described below may be made from different materials depending on the desired physical properties. For example, the outer surface may be composed of materials to promote or inhibit tissue ingrowth. Optionally, the outer surface of the sheath may be coated, impregnated, or otherwise includes one or more drugs and/or compositions that promote or inhibit tissue ingrowth around the sheath. Materials designed to promote tissue ingrowth include, but are not limited to, Polyester velour fabric manufactured by Bard (e.g., Part Numbers 6107 and 6108) or a polypropylene mesh. It is noted that ePTFE of different pore sizes can induce ingrowth. Tissue ingrowth into the sheath provides a tissue capsule in which the implanted system is secured within. The capsule protects surrounding tissue from possible damage from the implanted system as well as preventing tissue impingement upon the components of the implanted system. Additionally, the capsule allows the components and parts of the implant system to be easily accessed for maintenance and/or service since the components are located within the fibrous capsule. If a sheath is configured to include tissue ingrowth, then tissue is attached to the sheath with the benefit being no relative motion between the implant and tissue. Thus, all relative motion is between the moving implant and inner diameter of the sheath.

Materials that inhibit tissue ingrowth include, but are not limited to, expanded polytetrafluoroethylene (ePTFE) supplied by Zeus or International Polymer Engineering, polytetrafluoroethylene (PTFE) supplied by Bard (e.g., Bard p/n 3109, 31 12, or 6108), polyetheretherketone (PEEK) supplied by Secant Medical, silicone supplied by Accusil, Limteck, Promed Molded Products, Silicone Speciality Fabricators, TYGON® (e.g., 80 shore A material), or thermo-plastic polymers such as, but not limited to, C-FLEX®. Sheath embodiments made from one or more of the above-listed materials encourage tissue surrounding the sheath to form a non-adherent pseudo-capsule around the sheath. The pseudo-capsule isolates and stabilizes the implanted system thereby allowing easy access to the system while preventing tissue impingement upon the components of the implanted system.

In those sheath embodiments formed from ePTFE, the length change of the link or absorber element of the implanted system due to the flexion of the members to which it is attached, is taken up by the sheath material. It has been discovered that ePTFE is a preferred material for the sheath because it has good flexing and bending characteristics without kinking, it accommodates twisting, lengthening and shortening and it is a soft material that presents a soft surface to the surrounding tissues. Expanded PTFE has a microstructure having roughly parallel-running clumps of material (i.e., nodes) with perpendicular fibers (i.e., fibrils) connecting the nodes together. The spacing between the nodes and the fibrils of the ePTFE sheath allows for significant elongation and compression of the material (via stretching and compression of the fibrils) without adverse impact on the shape (e.g., inner or outer diameter) of the sheath. Additionally, the ability of the sheath to contract and expand allows the sheath to place a low tensile/compressive load on the moving link or absorber element of the implanted system.

According to one embodiment, a sheath made from ePTFE has an internodal distance of 25 microns. The low internodal distance has increased lubricity and radial strength as compared to materials having a high internodal distance. The low internodal distance of the material limits tissue ingrowth into the outer diameter of the sheath. In an alternate embodiment, the ePTFE has an internodal distance of 50 microns. The high internodal distance has decreased lubricity and increase porosity as compared to material having a low internodal distance. The high internodal distance has more tissue ingrowth (e.g., tissue penetrates wall). In yet another embodiment, one embodiment of a sheath includes a main body having a low internodal distance (e.g., 25 microns) that covers the absorber elements of the system, and end portions having a high internodal distance (e.g., 50 microns) that covers the base components.

According to one embodiment, the outer surface is made from a single type of material. In other embodiments, the outer surface is made from a plurality of materials. For example, the main body of the sheath is made of ePTFE, and the ends of sheath are made of PTFE. In this embodiment, the PTFE ends may be sutured to the ePTFE main body. Alternatively, the PTFE ends may be fused (or sintered) with the ePTFE main body.

Alternatively, the various embodiments of the sheath shown in FIGS. 1A-5B as well as those described below can be composed of a plurality of layers. In one embodiment, the sheath includes an outer layer that promotes or inhibits tissue ingrowth and one or more inner layers. The inner layer may be composed of a silicone sleeve, silicone foam layer, or a hydrogel. The silicone layer is used for padding in some embodiments. In another embodiment, the ends of the silicone layer are shaped to provide better fit of the sheath onto the base component. In another embodiment, the sheath includes an outer layer, a middle layer composed of a silicone layer, and an inner layer composed of ePTFE or PTFE. The inner layer may be coated with a lubricious coating (or the inner layer is made from materials having lubricious properties) that facilitate the movement one or more components of the energy absorbing system 44 within the sheath without binding, pinching, or otherwise limiting movement of the system within the sheath.

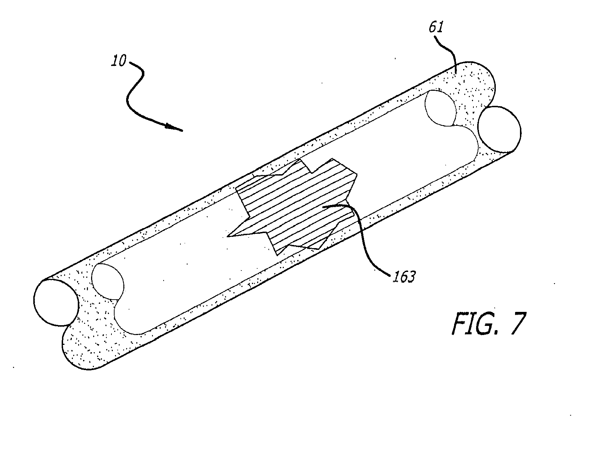

In one particular embodiment, a sheath 10 includes two separate layers 61, 63 as shown in FIG. 7. As shown in FIG. 7, the outer layer 61 has a larger diameter relative to the link or absorber (not shown) and the inner sheath 63. According to one embodiment, the outer layer 61 may be coated with material to cause or inhibit tissue ingrowth. The outer surface of the inner layer 63 may also include a lubricious coating thereby providing a low-friction surface between the inner layer and outer layer 61. Optionally, the inner surface of the inner layer 63 may also include a lubricious coating, thereby minimizing any pinching or undue friction between the link or absorber (not shown) and the inner layer.

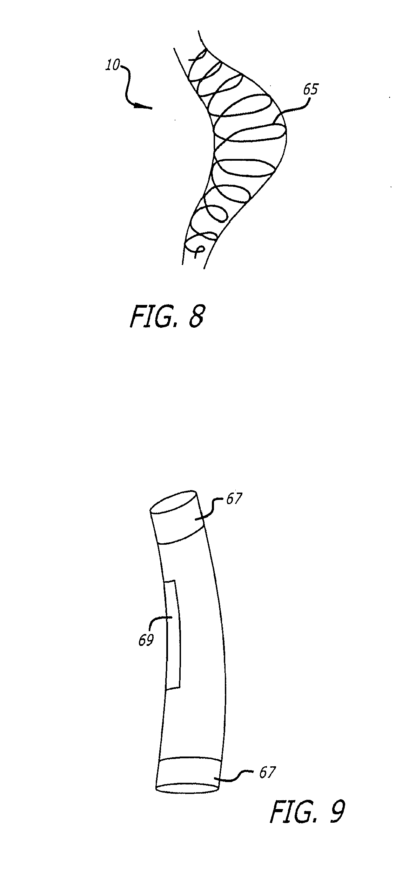

Optionally, the sheath 10 includes an internal support 65 as shown in FIG. 8. In one approach, the internal support 65 is one or more wires wound helically about the inner diameter of the sheath 10. In another approach, a wired lattice or metal lattice is coupled to the inner diameter of the sheath by sintering, gluing, or other means for securing two surfaces together known or developed in the art. The internal support 65 for the sheath prevents kinking or bunching of the sheath 10, which may interfere with the operation of the underlying link or absorber element (not shown).

FIG. 9 illustrates another embodiment of the sheath 10 having variable wall thickness. As shown in FIG. 9, the ends 67 of the sheath 10 are thickened to facilitate mounting of the sheath 10 to a base component (not shown) mounted on the bone. Additionally, areas of flexion or compression 69 may be thinner as shown in FIG. 9. The desired movements and response to forces may be achieved by providing a wall having variable thickness.

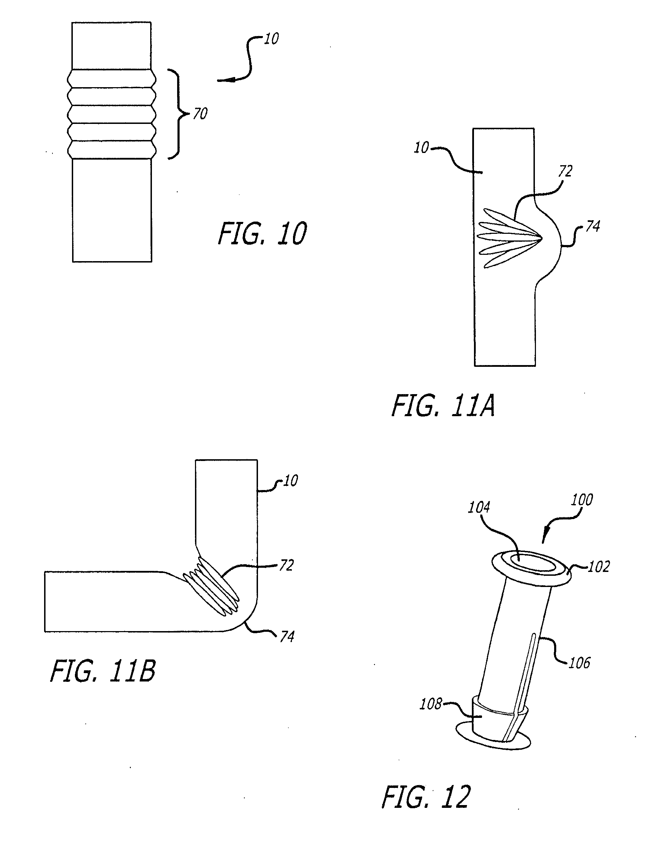

In another embodiment, as shown in FIG. 10, the sheath 10 includes regions that are folded in an accordion-like fashion. These folded regions of the sheath provide zones of flexion that prevent kinking, binding, or ovalizing of the sheath that may occur during the full range of motion of the components that comprise an extra-articular implantable link or mechanical energy absorbing system.

In yet another embodiment, the sheath 10 includes a biasing member 72 such as, but not limited to, a spring. The biasing member 72 is fixed at the pivot point 74 of the sheath as shown in FIGS. 11A-B. The biasing member 72 is fixed to or otherwise integrated into the walls of the sheath 10. As shown in FIG. 11A, the sheath 10 is shown in a default (or unflexed) position, and the biasing member 72 is compressed at one side of the member and expanded at the opposite side of the member. Alternatively, the biasing member 72 is positioned at an area of flexion, but the biasing member is not compressed. FIG. 11B illustrates the sheath 10 in a flexed position where the biasing member 72 is compressed and the area of flexion does not kink, bind, or otherwise deform when the sheath (and the underlying extra-articular implantable link or mechanical energy absorbing system) moves through the full range of motion.

As stated, the various disclosed embodiments of the sheaths are affixed to one or more components of the extra-articular implantable link or a mechanical energy absorbing system. According to one embodiment, the sheath is coupled to the base component of the implantable system. In another embodiment, the sheath is coupled to the mount of the implantable system. The various embodiments of the sheath disclosed herein include attachment structure for coupling the sheath to the implantable system. The attachment structure is configured to securely couple the sheath to a component of the implantable system while resisting any decoupling of the sheath from the component due to expansion and/or compression of the system.

For example, FIGS. 1A-D illustrates one embodiment of an attachment mechanism. As shown in the figures, the attachment mechanism is composed of reinforced areas 18 at the ends of the sheath. According to one embodiment, the reinforced areas 18 are composed of a disc of ePTFE (or PTFE) that is sintered to the sheath 10. In one embodiment, the reinforced area 18 is 10 mm in diameter, but those skilled in the art will appreciate that the reinforced area may have any size. As shown in FIGS. 1A-D, an opening 16 is located (e.g., centered, off-set) on the reinforced area 18 and is sized to receive a fastening member (not shown). The fastening member may be a screw, rivet, split-pin, or other fastening means known or developed in the art. The fastening member securely attaches the sheath to the base component (or the mounts) in a manner such that the extension and compression of the sheath does not cause the sheath to detach from the base component.

According to one embodiment, the fastening member used to secure the sheath 10 of FIGS. 1A-D is a retention pin 100 (See FIG. 12). As shown, the retention pin 100 includes a circular-shaped head 102 with a bore 104 provided therein. The body 106 of the retention pin 100 is generally cylindrical in shape. As shown in FIG. 12, the distal portion of the body 106 is divided into quadrants. Each quadrant of the body 106 includes barbs 108 on the outer circumference. In other embodiments, the body may be divided into two halves. Other embodiments of the retention pin (not shown) may not include barbs on the outer circumference. The retention pin 100 is made of a material such as, but not limited to, stainless steel or plastic.

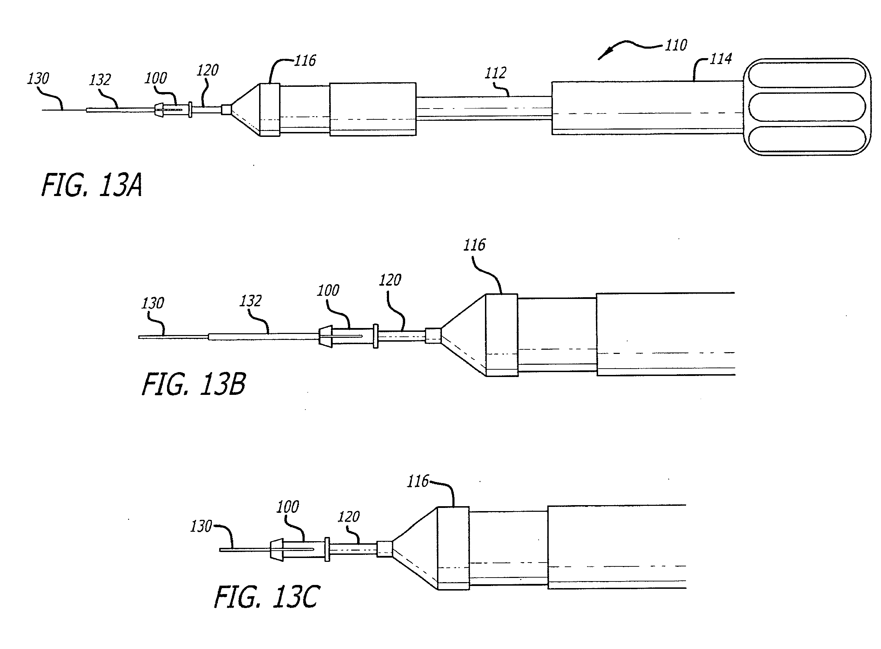

FIG. 13A illustrates a device 110 used to insert the retention pin 100 through the sheath 10 (e.g., as shown in FIGS. 1A-D) in order to secure the sheath to a component of the extra-articular implantable mechanical energy absorbing system. The device 110 has a generally cylindrical body 112 that includes a handle 114 at one end of the device. According to one approach, the handle 114 is approximately 76 mm in length, and the handle has a first diameter of approximately 12.5 mm and a second diameter (at the tip of the handle) of approximately 22 mm. In alternate embodiments, the handle 112 has a uniform diameter.

The device 110 includes a guide wire 130 at the end opposite the handle 114. The guide wire 130 may be made of Nickel Titanium (Nitinol) or any other super-elastic material that is able to withstand some deformation when a load is applied to the guide wire and allows the guide wire to return to its original shape when the load is removed. The guide wire 130 allows a user to locate an opening on a component of the implanted system in which the retention pin will be inserted. The guide wire 130 is coupled to a shaft 132 having an outer diameter that is smaller than the inner diameter of the retention pin 100. As a result, the retention pin 100 is slidable along the length of the shaft 132. The shaft 132, in turn, is coupled to the cylindrical body 112 of the device 110.

The device 110 includes a moveable handle 116 that is slidably coupled to the body 112. According to one embodiment, the moveable handle 116 is approximately 45 mm in length and approximately 12.75 mm in diameter. The moveable handle 116 slides relative to the cylindrical body 112 such that the moveable handle may be extended away or contracted toward the handle 114. The moveable handle 116 has approximately 17.5 mm of travel along the cylindrical body 112. However, as those skilled in the art will appreciate, the device 110 may be designed to have any travel distance for the moveable handle 116. Further, the device 110 has an approximately 9.5 mm gap between the moveable handle 116 and the first handle 114 in a contracted position. Any gap size or a total lack of a gap between the handles 114, 116 is contemplated in other embodiments of the device.

As shown in FIGS. 13B-C, the distal end of the moveable handle 116 is coupled to a cylindrical tube 120. The cylindrical tube 120 has an inner diameter that is larger than the outer diameter of the shaft 132 of the device 110. FIG. 13B illustrates the device 110 in a contracted position where the retention pin 100 is resting against the cylindrical tube 120 at the end of the shaft 132 closest to the handle 114. The cylindrical tube 120 moves the retention pin 100 along the shaft 132 as the moveable handle 116 is extended away from the handle 114. FIG. 13C illustrates the location of the retention pin 100 at the end of the shaft 132 and prior to the pin being inserted into an opening of a component (e.g., base component) of the extra-articular implantable mechanical energy absorbing system. Upon further advancement along the guide wire 130, the pin 100 can be fixedly inserted, for example, through the opening 16 of the reinforcement area 18 of a sheath (See FIGS. 1C and D) to thereby attach the sheath to an implantable device.

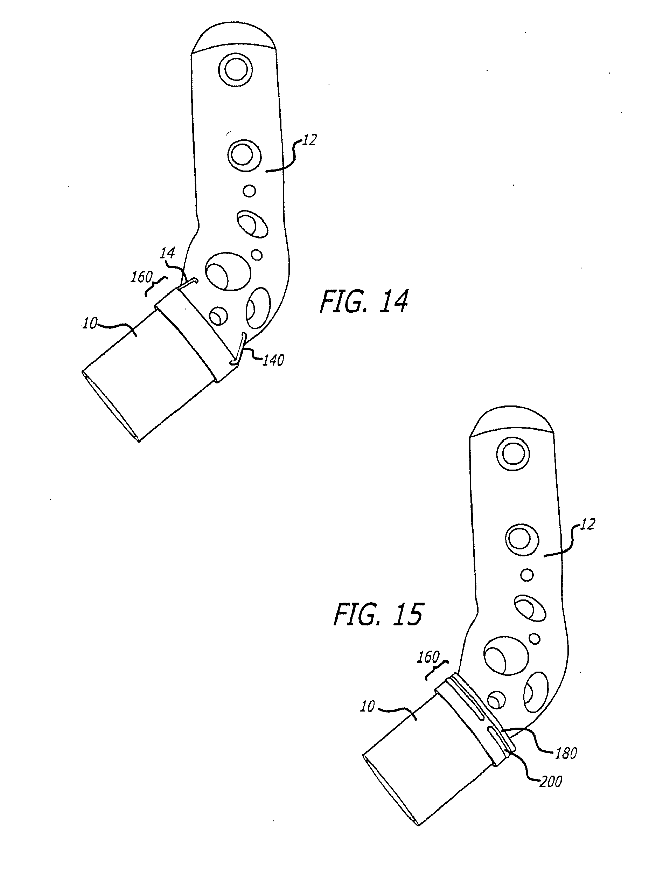

FIG. 14 illustrates another embodiment of an attachment structures used to couple the sheath 10 to a base component 12. In this approach, the attachment structures is a wire 140 that is wrapped around the circumference of the sheath 10 and the ends of the wire exit through holes on the sheath. The ends of the wire 140 are inserted into points of attachment on the sides of the base component 12. According to one embodiment, the point of attachment is an opening connected to a groove sized to receive the ends of the wire 140. The groove secures the wire 140 in a fixed position and also allows the sheath to maintain a low profile. Additionally, the grooves facilitate the installation of the sheath 10 onto the base component 12. In an alternate embodiment, the base component 12 only includes through holes for securing the sheath 10 to the base component. As shown in FIG. 14, a portion of the sheath wall 160 is everted and secured onto the outer diameter of the sheath 10. Alternatively, the sheath wall is inverted into the inner diameter of the sheath 10.

FIG. 15 illustrates another embodiment of an attachment approach used to couple the sheath 10 to a base component 12. As shown in FIG. 15 a portion of the sheath wall 160 is everted and secured onto the outer diameter of the sheath 10. Alternatively, the sheath wall 160 is inverted into the inner diameter of the sheath 10. A closed ring 180 is held in the space formed by the everted or inverted sheath wall 160. The closed ring 180 has a circumference that allows the ring to fit over the base component 12. Additionally, the closed ring 180 is a solid ring that preserves the edge strength of the sheath 10. A C-shaped ring 200 is also positioned over the sheath 10 as shown in FIG. 15. The C-shaped ring 200 secures the sheath 10 to the base component 12. The C-shape ring 200 is expanded, positioned, and then released to secure the sheath 10 to the base component 12. According to one embodiment, the base component 12 includes a circumferential groove (not shown) at the end of the base component. The circumferential groove secures the C-shaped ring 200 onto the base component 12 and ensures that the C-shaped ring 200 does not slip off the base component.

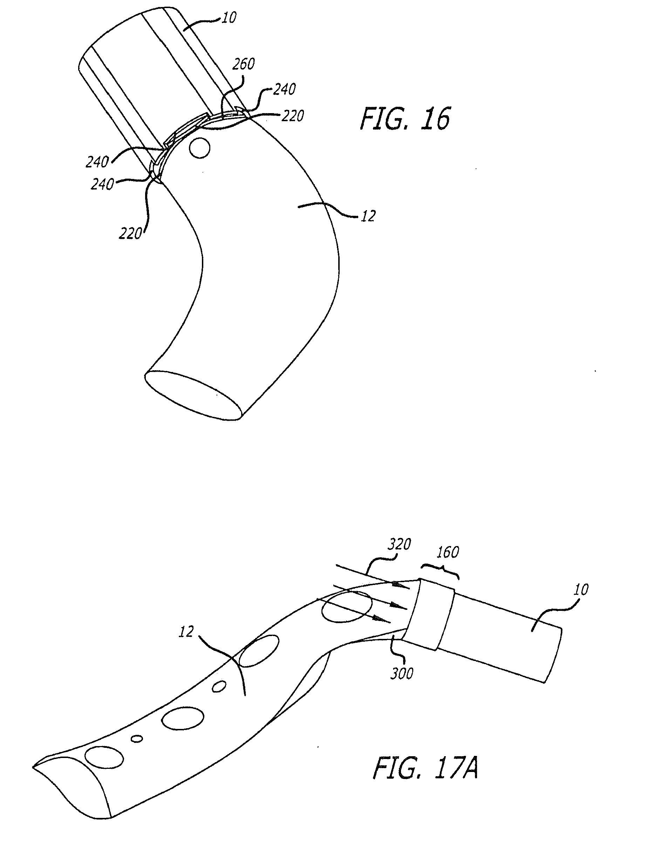

Turning to FIG. 16, another embodiment of a sheath 10 coupled to the base component 12 is illustrated. As shown, the edge of the sheath 10 includes three openings 240, and a wire 220 is exposed through the openings 220. A portion of the sheath wall (not shown) is inverted and secured onto the inner diameter of the sheath 10. Alternatively, the sheath wall is everted onto the outer diameter of the sheath 10. The exposed portion of the wire 220 are positioned with a circumferential groove 260 located on the base component 12.

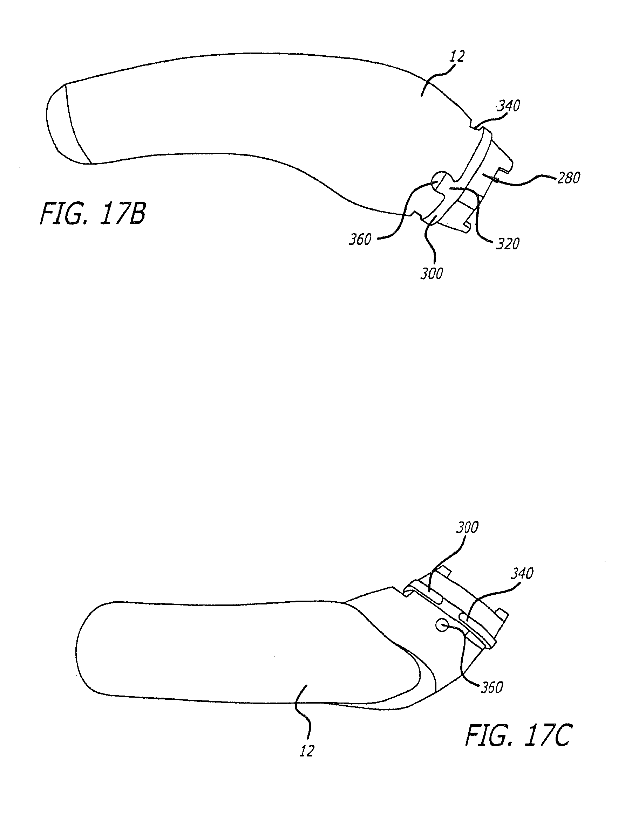

FIGS. 17A-17C illustrate yet another approach of attaching a sheath 10 to the base component 12. As shown in FIG. 17A, a portion of the sheath wall 160 is everted and secured onto the outer surface of the sheath 10. Alternatively, the sheath wall 160 is inverted into the inner surface of the sheath 10. The folded portion of the sheath wall 160 defines a space for a three-point snap ring 280. The three-point snap ring 280 includes hook arms 300, 320, 340 for coupling the sheath 10 to holes 360 or slots provided on the base component 12. FIGS. 17B-C show the three-point snap ring 280 coupled to the base component 12 without the sheath 10. The hook arms 300, 340 of the three-point snap ring 280 are coupled to the sides of the base component 12, and the central hook arm 320 is coupled to an opening 360 on the surface of the base component. As shown in FIG. 17C, the side hook arms 300, 340 wrap around the sides of the base component 12.

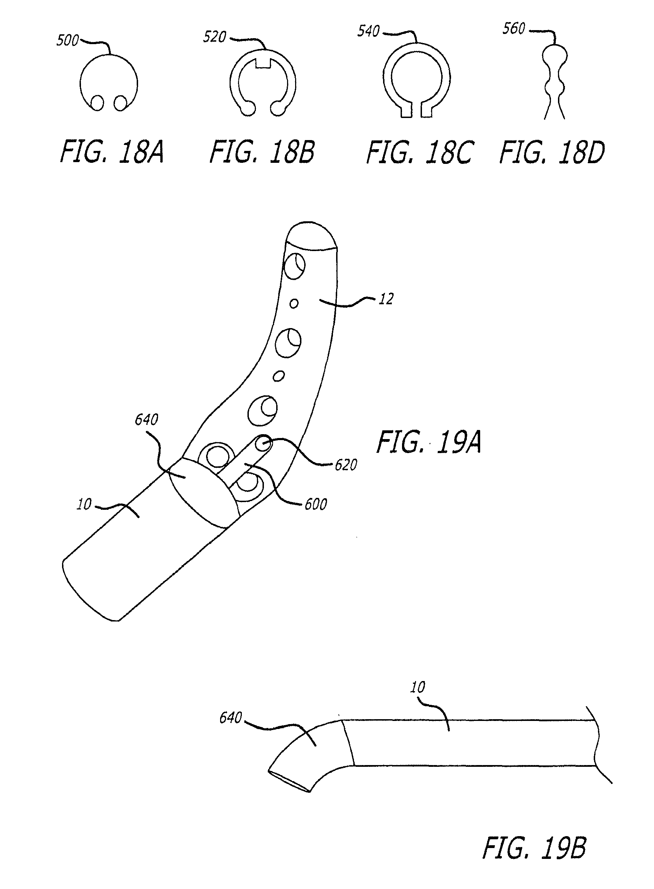

In other embodiments, the three-point snap ring 280 may be substituted for different types of clips. These clips include, but are not limited to, a housing ring 500 as shown in FIG. 18A, an E-clip 520 as shown in FIG. 18B, a snap ring 540 as shown in FIG. 18C, or a pin clip 560 as shown in FIG. 18D. The clips 500, 520, 540 and 560 may be made of an elastic alloy and coated with a silicone or other materials to form a softer surface against the sheath material.

FIG. 19A illustrates yet another embodiment of sheath 10 coupled to a base component 12. The sheath 10 includes a tab 600 that may be secured to the base component 12 via a screw, snap, or any other fastening means known or developed in the art. Additionally, the sheath 10 includes an angled surface 640. The end of the sheath 10 is cut at an angle so that it contours the shape of the base component 12 as shown in FIG. 19B.

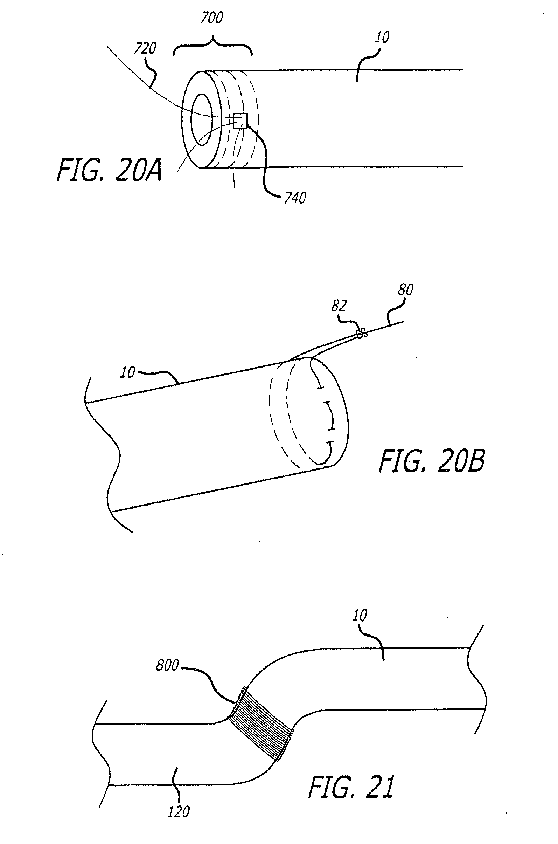

With reference to FIG. 20A, an embodiment of a sheath 10 having a purse-string attachment 700 is shown. The purse string attachment 700 includes a portion of the sheath wall (not shown) that is everted and secured onto the inner diameter of the sheath 10. Alternatively, a portion of the sheath wall is inverted onto the outer diameter of the sheath 10. The folded portion of the sheath 10 defines a space within the sheath in which a purse-string suture 720 resides. The suture 720 is placed radially around portions of the sheath. An opening 740 allows the suture 720 to be pulled in tension. As the suture is pulled, pleats form upon radial compression of the sheath. The suture 720 is then knotted, thereby securing the sheath 10 to the base component 12. According to one embodiment, the base component 12 includes a circumferential slot (not shown) on the edge of the base component to capture the sheath 10.

As shown in FIG. 20B, another embodiment of a sheath 160 can have at least one suture 800 provided at the ends of the sheath. The suture 800 is sewn around the circumference of the sheath 160 and includes a sliding knot 820 such as, but not limited to, a Tennessee slider or SMC knot. An arthroscopic knot pusher or a suture cutter (not shown) may be used to place the knot 820, tighten the suture, and secure the knot against the surface of the sheath 160.

A sheath 10 utilizing a wound suture 800 to secure the sheath to a base component 12 is also contemplated (See FIG. 21). The suture 800 is wound in tension around the sheath 10 and the base component 12. According to one embodiment, the base component 12 may include a slot (not shown) to ensure a low profile of the sheath 10 and suture 800 when coupled to the base component 12. In another embodiment, the suture 800 is sealed with or encapsulated in silicone.

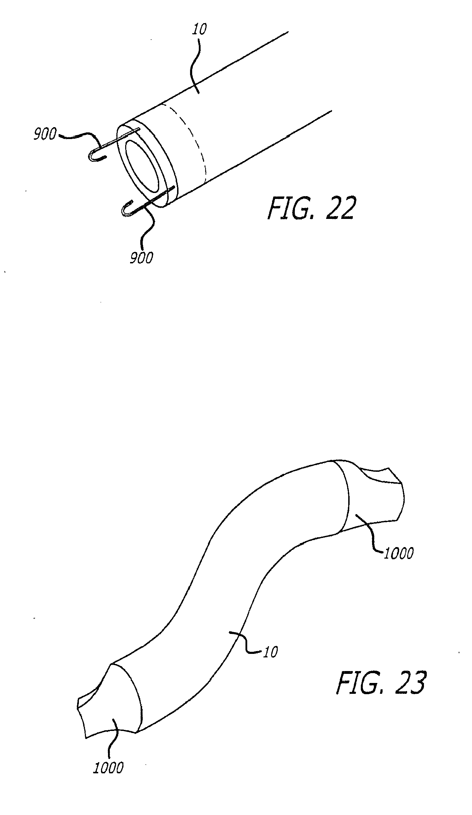

A sheath 10 may also include a plurality of hooks 900 provided on the ends of the sheath. As shown in FIG. 22, a portion of the sheath wall (not shown) is folded (e.g., inverted or everted) onto the inner diameter of the sheath 10. The hooks 900 are embedded, sewn, molded, or otherwise secured to the end of the sheath 10. The hooks 900 are positioned on the sides of the sheath 10 and engage grooves, indentations, holes, or other mating features on the sides of the base component to capture the hooks. The hooks 900 are positioned on the sides of the sheath 10 to ensure a low profile of the sheath when mounted to the base component. In one embodiment, the hooks 900 are coated with silicone or other padding material.

As depicted in FIG 23, another embodiment of a sheath 10 having preformed end connectors 1000 is contemplated. The geometry of the end connectors 1000 matches the base component geometry. In one embodiment, the end connector 1000 is molded onto the sheath 10. The end connector 1000 may be force-fitted or snap-fitted onto the base component.

Alternatively, the ends of the sheath are shaped or annealed to match the unique contoured shapes of the base component of the implanted system. The annealed ends maintain the soft structure of the material (e.g., ePTFE or PTFE).

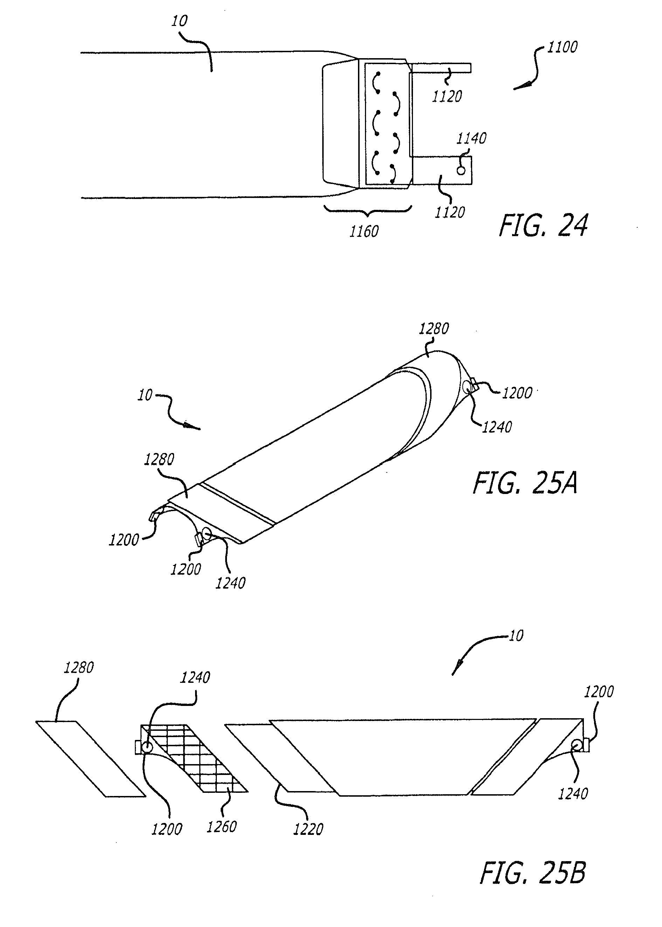

FIG. 24 illustrates an embodiment of a sheath 10 having a clip 1100 having one or more lock tabs 1120. The lock tabs 1120 include one or more openings 1140 that allow the clip 1100 to be screwed or otherwise fastened to the base component (not shown). The clip 1100 is made from a relatively inelastic metal. Accordingly, the clip 1100 is sized to mate with the base component (not shown). Alternately, the clip 1100 is made from an elastic metal or alloy that allows the clip to be expanded and snapped into place. The main body of the clip 1100 includes a plurality of openings for suturing the clip 1100 to the sheath 10. The clip 1100 also may be fused to the end of the sheath 10. As shown in FIG. 23, the clip 1100 is sutured to an end portion 1160, which in turn is coupled to the main body of the sheath 10. According to one embodiment, the main body of the sheath 10 is made of ePTFE, and end portions 1160 of the sheath are made from PTFE fabric. The PTFE end portions of the sheath 10 may be fused or sutured to the ePTFE main body. The PTFE fabric acts as a transition material between the clip 1100 thereby minimizing cold compression of the ePTFE material.

FIGS. 25A and B depict one embodiment of a sheath 10 having hooks 1200 provided at the ends of the sheath. The hooks 1200 fit within depressions or other shaped mating features in the base component (not shown). The hooks 1200 hold the sheath 10 in place when the sheath is under tension. As shown, the ends 1220 of the sheath 10 are cut at an angle so that the sheath does not need to be worked under the base component during installation. The sheath 10 also includes holes 1240 for an instrument for adjusting and manipulating the sheath.

The clip body 1260 (having the hooks 1200) is a stiff polymer, metal or metal alloy having a mesh or a lattice-type body. According to one embodiment, the clip body 1260 is an incomplete ring that is open at the bottom of the body. An incomplete ring simplifies the manufacturing process as the body may be photo-etched from a flat sheet of material and bent to the final shape. Alternatively, the clip body 1260 may be a complete ring. The clip body 1260 is positioned over the end 1220 of the main body of the sheath 10. An outer sheath 1280 is placed over the end 1220 of the sheath and the clip body 1260. The outside sheath material 1280 is then sintered or otherwise coupled to the main body of the sheath 10, thereby sandwiching the clip body 1260 between the outer sheath material and the ends of the main body.

In one embodiment, the clip body 1260 is approximately 0.2 mm thick. The clip body 1260 is relatively thin so that the ring has a combination of flexibility, rigidity, and a low profile. The sheath body 10 and the outer sheath material 1280 are approximately 0.6 mm thick. As those skilled in the art will appreciate, the thickness of the clip body 1260, outer sheath 1280 and main body may be varied to achieve different sheath profiles and characteristics (e.g., flexibility and/or rigidity).

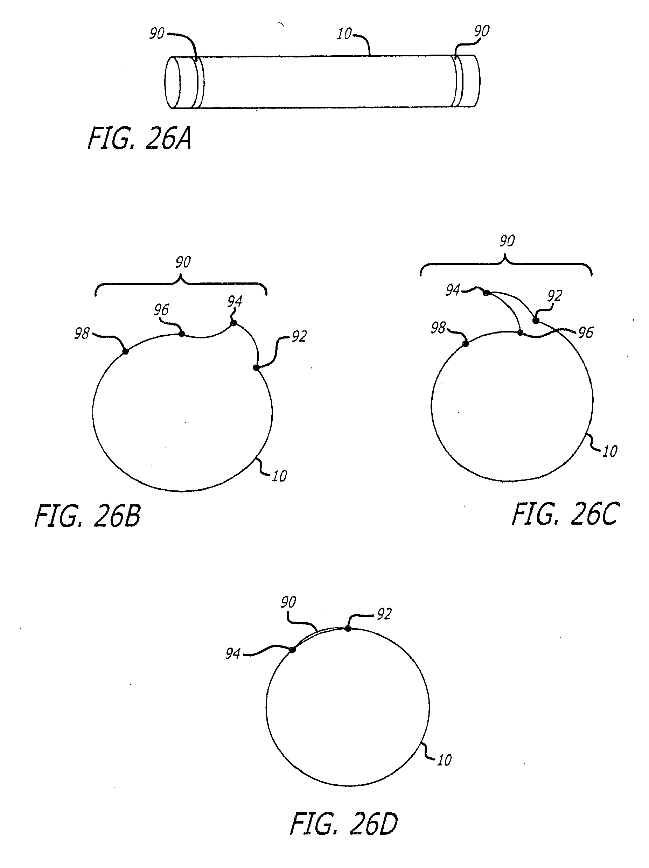

A sheath can also be equipped with quick-attach clips provided at the ends of the sheath as shown in FIG. 26A. FIG. 26B shows a cross-sectional view of the sheath 10 and the clip 90. The clip 90 is composed of a plurality of low-profile hinges 92, 94, 96, 98 positioned on a portion of the circumference of the sheath. As shown in FIG. 26B, the clip 92 is shown in the open position (i.e., a radially expanded position). The clip 92 is shown in a semi-closed position with a first hinge 92 positioned above the third hinge 96 and a second hinge 94 positioned above the fourth hinge 98 (See FIG. 26C). FIG. 26D illustrates the clip 90 in the closed position (i.e., radially reduced position).

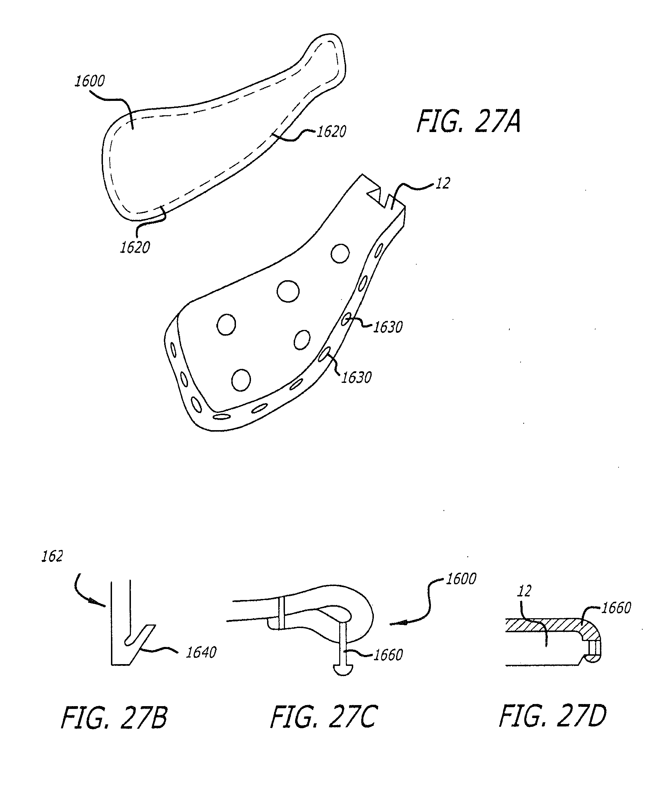

With reference now to FIG. 27A, one embodiment of a protective covering 1600 is configured to cover a base component 12. The protective covering 1600 is designed to provide cushioning to those base components 12 that are mounted to superficial bones (i.e., bones close to the surface of the skin) or have minimal fatty (or other) tissue, such as the tibia, that would cushion the base components. According to one embodiment, the protective covering 1600 is shaped to cover the upper surface of a base component. The protective covering 1600 has a generally low profile, and may also include one or more layers of cushioning. A plurality of fastening means 1620 are positioned around the perimeter of the sheath 1600. The fastening means 1620 may be hooks, clips, wires, loops, snaps, tabs, sutures, openings for screws, or other fastening means known or developed in the art. The base component 12 includes a plurality of openings 1630, eyelets, grooves, or ridges positioned on the sides (or on top) of the base component to accept the fastening means 1620 on the base component 12.

FIG. 27B is a cross-sectional view of one embodiment of a fastening means 1620 for coupling the covering 1600 to the base component 12. The fastening means 1620 is a hook 1640 that is secured to an inverted edge of the covering 1600. The hook 1620 is secured within an opening on the side of the base component 12. FIG. 27C illustrates another embodiment of a hook 1660 having a mushroom-shaped head fixed to an inverted edge. As shown in FIG. 27C, a suture 1670 secures the hook 1660 within the space formed by the inverted edge. FIG. 27D illustrates another embodiment the hook 1660 inserted into an opening on the side of the base component 12.

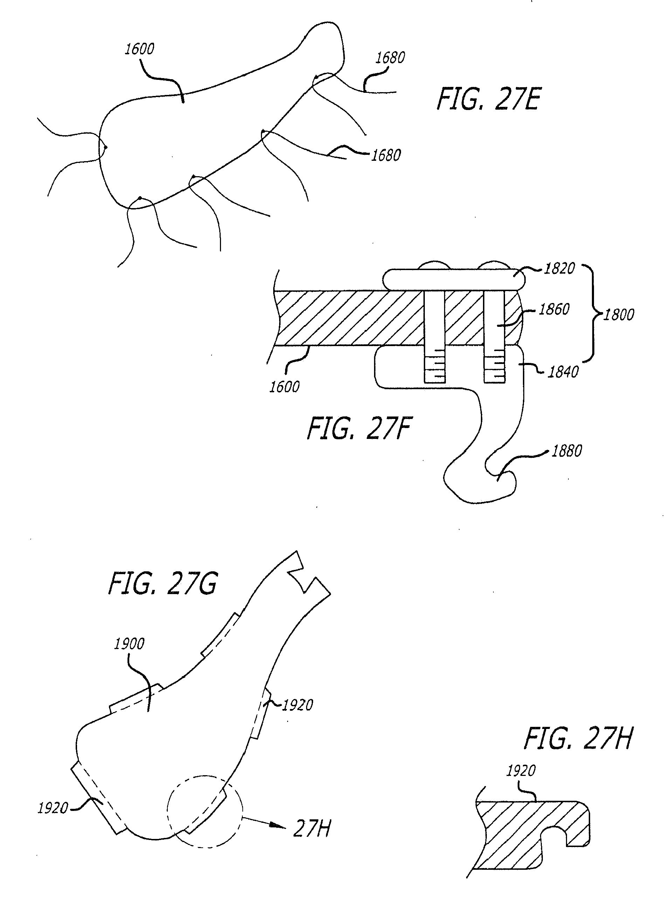

Turning to FIG. 27E, a covering 1600 can include sutures 1680 provided around the perimeter of the covering. The sutures 1680 are secured to the bottom surface of the covering 1600 via knots. The sutures 1680 may be secured (threaded through, wound around) corresponding eyelets 1690 on the base component 12.

Moreover, as shown in FIG. 27F an embodiment of a covering 1600 may have clips 1800 provided on the periphery of the covering. The clip 1800 includes a top plate 1820 that is coupled to a bottom plate 1840 with screws 1860 or other fastening means. The bottom plate 1840 also includes a hooking member 1880 for engaging a portion of the base component. The covering 1600 is secured between the top and bottom plates 1820, 1840.

In another approach (FIG. 27G-H), a protective covering 1900 can include tabs 1920 positioned about the perimeter of the covering. The tabs 1920 are integral with the covering (i.e., made from same material as covering 1900). Alternatively, the tabs 1920 may be separate components secured to the covering 1900. The tabs 1920 are foldable and inserted into a groove or opening on the base component. FIG. 27H shows one configuration of a tab 1920 having a generally hooked-shape that may be inserted into a groove, slot, or opening on the base component.

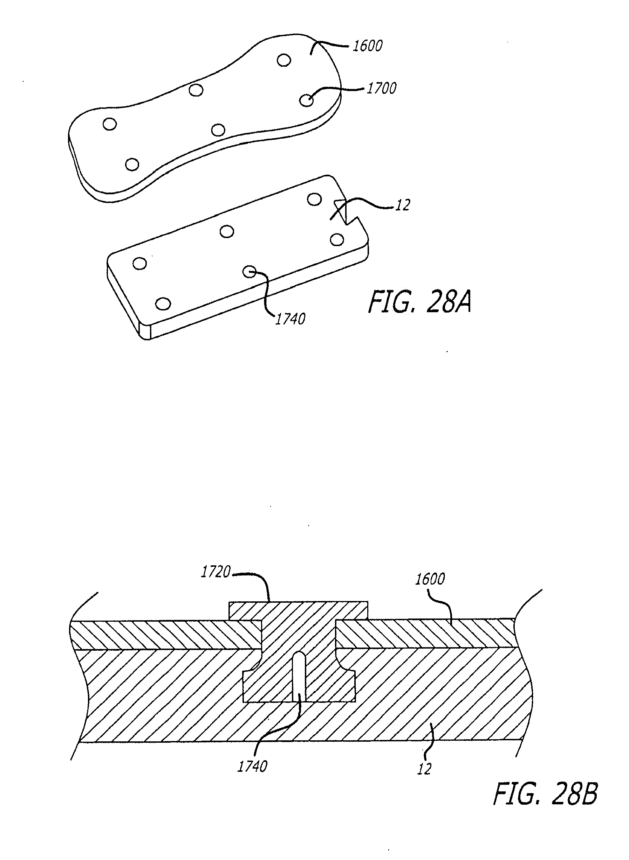

FIG. 28A illustrates an embodiment of a protective covering 1600 having holes 1700 on the surface of the sheath. The holes 1700 are designed to accept a plug 1720 to lock the covering 1600 onto the base component 12 (plug is inserted into openings 1740 on the base component) as shown in FIG. 28B.

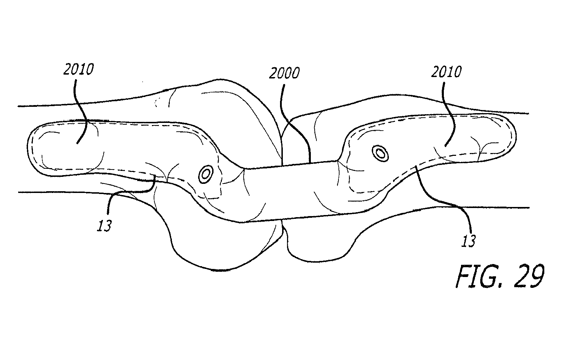

Turning now to FIG. 29, yet another approach to excluding the extra-articular energy absorbing system from surrounding tissue is disclosed. Here, the contemplated structure includes a sheath portion 2000 which is sized to cover the mid-section of the system as well as integrally formed protection covers 2010 sized and shaped to cover bases 12 (shown in phantom) attached to bone members.

Accordingly, the presently disclosed approaches to sheaths can be configured to protect tissue within an interventional site and exclude as is desired, various components of medical devices such as energy manipulating or other devices from surrounding tissue. The sheaths create spaces within the interventional area such that removal or adjustment of implanted devices can be more easily accomplished. Moreover, various approaches to useful materials and coatings have been disclosed as well as structure for attaching the sheaths within the interventional site. It is to be recognized that such features can be applied to any implanted medical or other device to achieve contemplated objectives.

The various embodiments described above are provided by way of illustration only and should not be construed to limit the claimed invention. Those skilled in the art will readily recognize various modifications and changes that may be made to the claimed invention without following the example embodiments and applications illustrated and described herein, and without departing from the true spirit and scope of the claimed invention, which is set forth in the following claims.

## Claims

1. A sheath for an extra-articular implantable mechanical energy absorbing system, the sheath comprising:
an elongated body having an inner diameter and an outer diameter, the elongated body having a first end and a second end;
a first attachment structure, the first attachment structure attaching the first end to a first component of the extra-articular implantable mechanical energy absorbing system; and
a second attachment structure, the second attachment structure attaching the second end to a second component of the extra-articular implantable mechanical energy absorbing system
wherein the sheath protects and excludes tissue surrounding the extra-articular implantable mechanical energy absorbing system from movement of the system by forming a capsule.

2. The sheath of claim 1, wherein the body is formed from ePTFE.

3. The sheath of claim 1, further comprising an internal support member fixed to the elongate body, wherein the internal support member has a generally helical shape spanning the length of the elongated body.

4. The sheath of claim 1, further comprising a cushioning layer coupled to the elongated body.

5. The sheath of claim 1, wherein the first and second attachment structure includes a molded end piece integral with the elongated body.

6. The sheath of claim 1, wherein the first and second attachment structure further comprises a wire loop embedded within the first and second ends of the sheath, wherein a first end and second ends of the wire loop exit a leading edge of the sheath, and wherein the first and second ends of the wire loop are hook-shaped.

7. The sheath of claim 1, wherein the first and second attachment structure further comprises a clip fixed to the first and second ends of the sheath, wherein the clip is shaped to engage a corresponding surface on the first and second components.

8. The sheath of claim 1, wherein the first and second attachment structure further comprises a plurality of hooks extending from the ends of the sheath, wherein the plurality of hooks engage an opening or surface of the first and second components.

9. A protective covering system for an extra-articular implantable mechanical energy absorbing system, the protective covering system comprising:
an extendable extra-articular energy absorbing system configured for extra-articular placement across a joint;
at least two base components configured to be fixed to bones on opposite sides of the joint and connected to the energy absorbing system; a protective covering body for covering the energy absorbing system, the protective covering body having an upper surface, a lower surface, and a perimeter, wherein the body is shaped to cover an upper surface of a at least one of the two base components; and
one or more coupling structures provided about the perimeter of the body, wherein the coupling structures secure the body to at least one of the two base components.
